# EUROPEAN PATENT APPLICATION

(11) **EP 3 034 127 A2**
(43) Date of publication of application: **22.06.2016**
(21) Application number: 15275266.3
(22) Date of filing: 17.12.2015
(51) Int. Cl.: A61M 25/10

(54) **ULTRASONICALLY VISIBLE MEDICAL BALLOON ASSEMBLY**

(30) Priority: 18.12.2014 GB 201422607
(71) Applicant: Cook Medical Technologies LLC, Bloomington, IN 47404 (US); Krasznai, Attila Gyorgy, 6416 EZ Heerlen (NL); Bouwman, Lee Hans, 6176 CG Spaubeek (NL)
(72) Inventor: Aggerholm, Steen, 4660 St. Heddinge (DK); Lysgaard, Thomas, 2680 Solroed Strand (DK); Krasznai, Attila Gyorgy, 6416EZ Heerlen (NL); Bouwman, Lee Hans, 6176CG Spaubeek (NL)
(74) Representative: Williams Powell

(57) **Abstract**

A balloon catheter assembly (10) includes a balloon structure (20) formed of an inner balloon (40) and an outer balloon (70) which entirely envelops the inner balloon (40). The inner balloon (40) can be filled with air or other echogenic fluid and inflates to a diameter substantially less than the inflated diameter of the outer balloon (70), typically to a diameter of no more than 50% of the inflated diameter of the outer balloon (70). The inner balloon (40) can be inflated with air or other echogenic fluid, enabling the balloon structure (20) to be visible under ultrasonic imaging. The inner balloon (40) is protected within the outer balloon (70) and is shorter than the outer balloon (70). The assembly (10) can be used for PTA procedures, for deploying an implantable medical device or for other medical applications.

## Description

### Technical Field

The present invention relates to a balloon catheter assembly, which may in example implementations be configured as a PTA balloon or a device deployment balloon.

### Background Art

Medical balloon assemblies are known in the art, for use for example in angioplasty procedures, for deploying implantable medical devices, and so on.

Medical balloons are typically made of a fine yet strong polymer material, such as polyethylene terephthalate (PETE), polyamide (Nylon) or the like, and are fitted to a balloon catheter, also typically made of a polymer material. These materials have the advantage of being biocompatible, being strong in use and also being flexible, which assists in the trackability of the apparatus, that is its ability to curve and bend through a patient's vasculature as it is fed endoluminally from a remote percutaneous entry point. A problem arises with such balloon catheters, however, in that they are difficult to see during imaging. Although it is possible to use imaging materials, such as contrast media, during the deployment of such balloons, the results are not always optimal. There is also a desire to avoid excessive use of MRI, although the nature of such balloon assemblies currently makes this the best visualization medium.

Similar problems arise with assemblies for deploying implantable medical devices, such as stents, stent grafts, filters and so on; which are conventionally deployed with the aid of MRI scanning.

Visualization of the balloon is particularly important in ensuring that the balloon is precisely positioned at the treatment site before it is deployed.

A number of techniques for enhancing the visibility of balloon catheters, as well as balloon catheter assemblies in general, have been disclosed in US-2014/0039358, US-2014/0024935, US-8,480,647, US-8,043,313, US-2006/0004323 and US-2013/0072792.

### Disclosure of the Invention

The present invention seeks to provide an improved a balloon catheter assembly, and in particular an assembly which has a structure and functionality enabling the assembly to be seen by ultrasonic imaging. The balloon assembly can be used for percutaneous transluminal angioplasty (PTA) procedures, for deploying implantable medical devices, or for other purposes.

According to an aspect of the present invention, there is provided a balloon catheter assembly including: a carrier catheter having a distal end and a proximal end, at least first and second lumens passing through the catheter; a balloon assembly including an inner inflatable balloon attached in fluid tight manner at the distal end of the carrier catheter, the inner balloon providing an inner chamber; and an outer inflatable balloon disposed concentrically over the inner balloon, the outer balloon being attached in fluid tight manner at the distal end of the catheter and providing an outer chamber; the first lumen of the catheter including an outlet fluidically coupled to the inner chamber, the second lumen of the catheter including an outlet fluidically coupled to the outer chamber, whereby the inner and outer balloons are independently inflatable; wherein the inner balloon has an inflated diameter no more than 50% of the inflated diameter of the outer balloon.

According to another aspect of the present invention, there is provided a balloon catheter assembly including: a carrier catheter having a distal end and a proximal end, and at least first and second lumens passing through the catheter; a balloon assembly including an inner inflatable balloon attached in fluid tight manner at the distal end of the carrier catheter, the inner balloon providing an inner chamber; and an outer inflatable balloon disposed concentrically over the inner balloon, the outer balloon being attached in fluid tight manner at the distal end of the catheter and providing an outer chamber; wherein the inner balloon has an inflated diameter no more than 50% of the inflated diameter of the outer balloon; the first lumen of the catheter including an outlet fluidically coupled to the inner chamber, the second lumen of the catheter including an outlet fluidically coupled to the outer chamber, whereby the inner and outer balloons are independently inflatable; and a source of echogenic fluid coupled to the first lumen, whereby the inner balloon is inflatable with said echogenic fluid.

The inner balloon can be filled with air, which is highly visible in ultrasonic imaging, thereby avoiding the need to rely on MRI scanning during a medical procedure. The fact that the inner balloon has an inflated diameter substantially less than the inflated diameter of the outer balloon means that the outer balloon does not expand fully, and preferably remains in a wrapped condition during the inflation of the inner balloon, thereby enabling the assembly to be moved within the vessel until it is precisely in the desired location. The outer balloon, which houses the inner balloon, can also act as a protection device to the inner balloon, enabling the inner balloon to be made less strong and therefore typically of thinner and/or more flexible material.

It is preferable that the outer balloon is wrapped over the inner balloon and wherein inflation of the inner balloon does not cause unwrapping of the outer balloon. The outer balloon may be wrapped over the inner balloon so as to have three to six or more folded balloon portions, or wings, wrapped around the carrier catheter.

Preferably, the inner balloon has an inflated diameter of no more than 2 millimetres, more preferably of no more than 1.5 millimetres. This ensures that the inflation of the inner balloon does not cause the outer balloon to unwrap completely, provides sufficient volume of air in a typical balloon assembly to be readily visible in ultrasonic imaging and also that a relatively small amount of air is used in the procedure.

Advantageously, the inner balloon is longitudinally spaced from the outer balloon on the carrier catheter. This ensures that the inner balloon can be wholly contained in the outer balloon and can ensure the assembly is wrappable and compressible to an optimum diameter for delivery.

Advantageously, the inner balloon is shorter than the outer balloon.

In the preferred embodiment, the inner balloon is longitudinally symmetrically disposed within the outer balloon, which enables the clinician to place the assembly precisely in the middle of the treatment site on the basis of the symmetry of the double balloon arrangement.

The assembly preferably includes a source of echogenic fluid, such as air, coupled to the first lumen, to inflate the inner balloon. In other embodiments the assembly is provided with a coupling for coupling a source of fluid to the inner balloon. The source of air may be a syringe.

The inner balloon may be made of a compliant material, in some embodiments of an elastomeric material. The inner balloon may be made of polyurethane or polyether block amide, for example.

There may be provided an implantable medical device carried on the balloon assembly, such as a stent or stent graft.

According to another aspect of the present invention, there is provided a balloon catheter assembly including: a carrier catheter having a distal end and a proximal end, at least first and second lumens passing through the catheter; a balloon assembly including an inner inflatable balloon attached in fluid tight manner at the distal end of the carrier catheter, the inner balloon providing an inner chamber; and an outer inflatable balloon disposed concentrically over the inner balloon, the outer balloon being attached in fluid tight manner at the distal end of the catheter and providing an outer chamber; the first lumen of the catheter including an outlet fluidically coupled to the inner chamber, the second lumen of the catheter including an outlet fluidically coupled to the outer chamber, whereby the inner and outer balloons are independently inflatable; wherein the outer balloon is wrapped over the inner balloon prior to inflation and wherein inflation of the inner balloon does not cause unwrapping of the outer balloon.

According to another aspect of the present invention, there is provided a balloon catheter assembly including: a carrier catheter having a distal end and a proximal end, at least first and second lumens passing through the catheter;
a balloon assembly including an inner inflatable balloon attached in fluid tight manner at the distal end of the carrier catheter, the inner balloon providing an inner chamber; and an outer inflatable balloon disposed concentrically over the inner balloon, the outer balloon being attached in fluid tight manner at the distal end of the catheter and providing an outer chamber; the first lumen of the catheter including an outlet fluidically coupled to the inner chamber, the second lumen of the catheter including an outlet fluidically coupled to the outer chamber, whereby the inner and outer balloons are independently inflatable; and a source of air connected to the first catheter lumen for inflating the inner balloon with air.

Other features and advantages of the teachings herein will become apparent from the specific description which follows.

### Brief Description of the Drawings

Embodiments of the present invention are described below, by way of example only, with reference to the accompanying drawings, in which:
Figure 1 is a schematic diagram of a balloon catheter assembly according to an embodiment of the invention;
Figure 2 is a side elevational view of the distal end of the assembly of Figure 1 with the balloons of the assembly shown in an inflated condition;
Figure 3 is a transverse cross-sectional view of the catheter of the assembly of Figure 1;
Figure 4 is a side elevational view of the distal end of the assembly of Figure 1 with the inner balloon in an inflated condition and the outer balloon uninflated;
Figure 5 is a transverse cross-sectional view of the balloons of the assembly of Figure 4 with the inner balloon in an inflated condition and the outer balloon still wrapped over the inner balloon; and
Figure 6 is a side elevational view of the distal end of a balloon catheter assembly according to an embodiment of the invention with a stent mounted on the balloons of the assembly.

### Description of the Preferred Embodiments

It is to be understood that the drawings are schematic only and not to scale. They are intended to depict the major components of the devices taught herein and minor or ancillary components are not shown for the sake of clarity. The skilled person will be well acquainted with the typical dimensions and proportions suitable for the components and devices shown in the drawings, particularly having regard to the accompanying description.

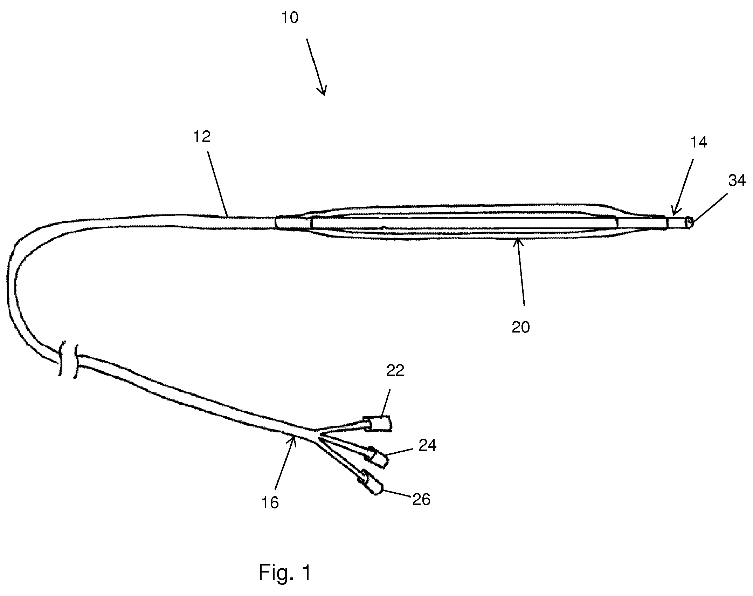

Referring first to Figure 1, this shows in schematic form an embodiment of balloon catheter assembly 10 according to the teachings herein. The assembly 10 includes an elongate catheter 12 having a distal end 14 and a proximal end 16. The catheter 12 is of a length such that the proximal end 14 can be positioned endoluminally at a treatment site in a vessel of a patient with the proximal end 16 remaining outside the patient. Typically, the catheter 12 may length from a few tens of centimetres to one metre or even longer, as required.

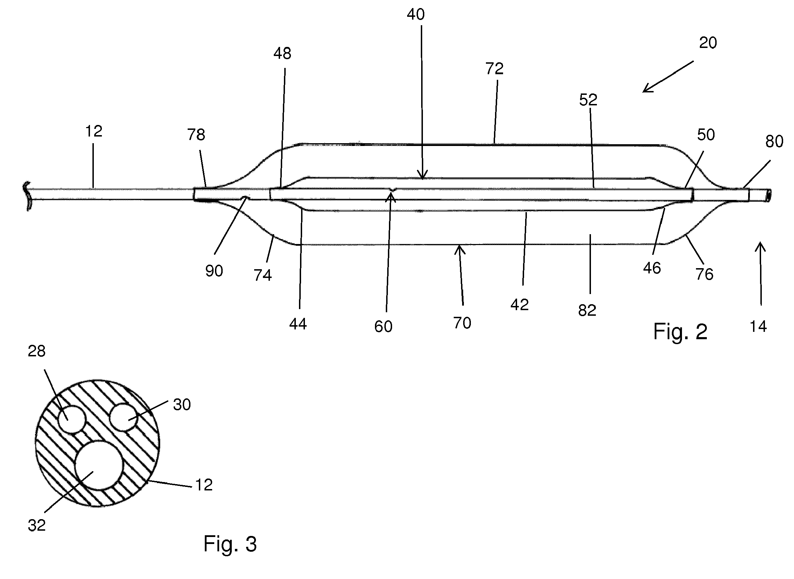

By the distal end 14 of the catheter 12 there is provided a balloon structure 20, described in further detail below. At its proximal end 16 the catheter 12 includes three feed ports 22, 24, 26 which feed respectively into associated lumens 28, 30, 32 (shown in Figure 3) which extend through the catheter 12. A central lumen 32 extends for the whole of the length of the catheter 12 to an exit point 34 at the distal tip 14 of the catheter 12 and provides for the passage of a guide wire (not shown) through the assembly 10, such that the assembly can be deployed "over-the-wire". The skilled person will appreciate that this lumen could be omitted where over-the-wire deployment is not necessary or desired.

The other lumens 28, 30 extend to the balloon structure 20 of the assembly 10 and are used to feed fluid into the two balloons of the structure 20, described in further detail below.

The catheter 12 can be made of any known and suitable material such as polyurethane, Nylon, silicone, polyethylene terephthalate and so on. The catheter 12 may be made solely of such materials but may also, as is common, include one or more strengthening elements within the structure of the catheter, such as a braided wire or coil. Such strengthening elements, particularly in larger diameter catheters, can assist in the pushability of the catheter through the patient's vasculature and in preventing kinking of the catheter.

Referring now to Figure 2, this shows in better detail the balloon structure 20 at the distal end 14 of the catheter 12. The balloon structure 20 includes an inner balloon 40 which in this embodiment has a conventional balloon shape, namely with a substantially cylindrical body portion 42, conical end portions 44 and 46, and proximal and distal necks 48 and 50 which are sealed in fluid-tight manner to the outer surface of the catheter 12. The inner balloon 40 provides a chamber 52 between its inside surface and the outer surface of the catheter 12. The catheter 12 includes an opening or port 60 in the portion of catheter located within the chamber 52, the port 60 being coupled fluidically to the lumen 28 within the catheter 12.

The structure 20 also includes an outer balloon 70, which in this embodiment also has a generally conventional shape, that is a generally cylindrical body portion 72 bounded by end cones 74 and 76 and having proximal and distal necks 78, 80 fixed in fluid-tight manner to the catheter 12. The outer balloon 70 has an internal chamber 82 which is principally the volume between the inner wall surface of the outer balloon 70 and the outer wall surface of the inner balloon 40.

The catheter 12 includes an opening or port 90 which is fluidically coupled to the lumen 30 of the catheter 12. The port 90 opens to the chamber 82 of the outer balloon 70.

The balloons 40 and 70 can be fixed to the catheter 12 in any known manner, for instance by heat bonding, by use of an adhesive or other bonding agent, and so on.

As will be seen in Figure 2, the inner balloon 40 is disposed wholly within the volume of the outer balloon 70 and such that the necks 48, 50 of the inner balloon 40 are also within that volume and specifically spaced from the necks 78, 80 of the outer balloon 70. Not only does this provide space for a convenient opening or port 90 in the catheter 12 to the chamber 82 of the outer balloon 70, but it also ensures that the whole assembly can be wrapped to a very small diameter for endoluminal delivery purposes. The inner balloon 40 is, as a result, shorter than the outer balloon 70. It is preferred that the inner balloon 40 is positioned symmetrically within the outer balloon 70, such that the gap between the proximal end 48 of the inner balloon 40 and the proximal end 78 of the outer balloon 70 is the same as the gap between the distal end 50 of the inner balloon 40 and the distal end 80 of the outer balloon 70. This symmetry, as explained below, ensures that the position of the outer balloon 70 can be accurately determined by viewing the location of the inner balloon 40.

As can be seen in Figure 2, the inner balloon 40, when inflated, has a much smaller diameter than the inflated diameter of the outer balloon 70, such that in practice it is the outer balloon 70 which effects the medical procedure, be it angioplasty or other opening of a vessel, deployment of a medical device, or the like. Advantageously, the inflated diameter of the inner balloon 40 is no more than 50% of the inflated diameter of the outer balloon 70, although in preferred embodiments the inner balloon may have an inflated diameter significantly smaller than this, for example 25%, 20% or even less, of the inflated diameter of the outer balloon 72.

In a practical embodiment, the inner balloon may have an inflated diameter of no more than 2 millimetres and in some embodiments of no more than 1.5 millimetres. For example, for a PTA balloon having an inflated diameter of 8 to 10 millimetres, the inner balloon may have an inflated diameter of 0.8 to 1.5 millimetres at most.

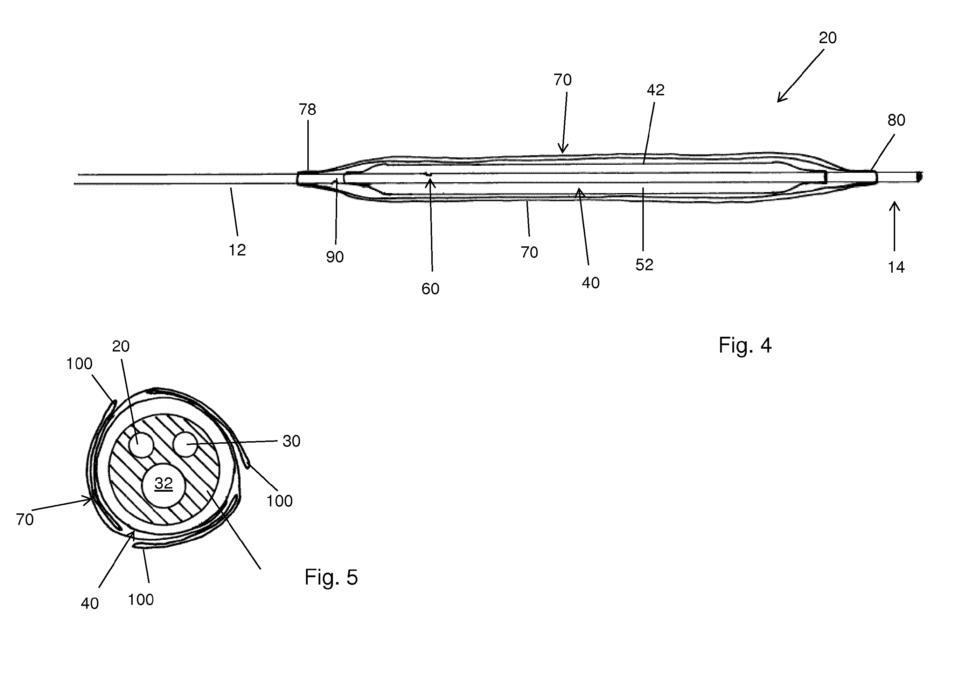

Referring now to Figures 4 and 5, these show the balloon structure 20 in a configuration in which the inner balloon 40 is inflated while the outer balloon 70 remains uninflated. As will be apparent particularly from Figure 5, in this condition the outer balloon 70 remains wrapped by folds 100 around the catheter 12 and inner balloon 40. That is, the inflated diameter of the inner balloon 40 is such as not to cause the outer balloon 70 to unwrap, until the latter is intentionally inflated with inflation fluid from the lumen 30. In practice, therefore, when the inner balloon 40 is inflated to its normal inflated diameter, the balloon structure 20 remains at a relatively small diameter, in practice at a diameter at which the outer balloon 70 is not pressed against the walls of the vessel, and mostly not in contact with the vessel. This allows the assembly 50 to be moved within the vessel for precise positioning. The inner balloon 40, in this inflated condition, will contain enough air or other echogenic fluid so as to be readily visible under ultrasonic imaging.

In use, the balloon catheter assembly 10 is fed endoluminally through a patient's vasculature from a remote percutaneous entry point with the balloon structure 20 in the deflated condition. This is typically done within an outer sheath as is known in the art. Once the distal end 14 of the assembly 10, and specifically the balloon structure 20, is positioned at the treatment site, the inner balloon 40 is inflated with air or other echogenic fluid by a suitable fluid supply, thereby to provide a volume of echogenic fluid within the chamber 52. This enables the structure 20 to be visualised by ultrasonic imaging. As a result, the location of the inner balloon 40 can be accurately determined during ultrasonic imaging. As a result of the preferred symmetrical disposition of the inner balloon 40 in the outer balloon 70, the position of the outer balloon can be directly established on visualising the inner balloon 40. This enables precise positioning of the balloon structure 20 at the desired treatment site. Once accurately positioned, the outer balloon 70 is inflated by injecting suitable inflation fluid, typically saline or other known solution, until it attains its deployed configuration and able to effect the medical treatment. The inner balloon 40 can be deflated when the outer balloon 70 is inflated or left in the inflated condition.

Until the outer balloon 70 is fully inflated, the assembly can therefore be moved proximally or distally in the vessel as necessary, since the inflation of the inner balloon is insufficient to cause unwrapping of the outer balloon 70. In this regard, it is also possible to deflate the inner balloon 40 after only the inner balloon 40 has been inflated, with such deflation allowing the outer balloon 70 to contract again as a result of the retention of the folds 100 in the wrapped balloon 70 and the natural resiliency of the material of the outer balloon 70.

It will be appreciated that the structure 20 is particularly compact in the longitudinal dimension. It also has the advantage of housing the inflatable balloon 40 entirely within the outer balloon 70. In this manner, should the inner balloon burst, the fluid used to inflate the inner balloon will remain within the chamber of the outer balloon 70 without escaping into the patient's blood stream. It should be noted, however, that the volume of air within the chamber 52 of the inner balloon 40 can be significantly less than a maximum safe threshold of air. Therefore, even were the air to escape from the inner balloon 40 this will not pose a health risk. The skilled person will in any event appreciate that the inner balloon 40 is not likely to burst as a result of the protection provided by the outer balloon 70. The arrangement also permits the use of a thin walled inner balloon 40, with the result that the structure 20 can be made more compact when wrapped to the catheter 12.

In practice, air can be fed into the chamber 52 of the inner balloon 40 by any suitable inflation source, a common syringe being an option.

The inner and outer balloons 40, 70 can be made of any known materials including, for example, a polyether block amide such as Pebax™, polyethylene terephthalate (PETE), a polyamide such as Nylon, or other suitable materials. The inner balloon can be made of the same material as the outer balloon or of compliant material, including polyurethane, silicone and the like.

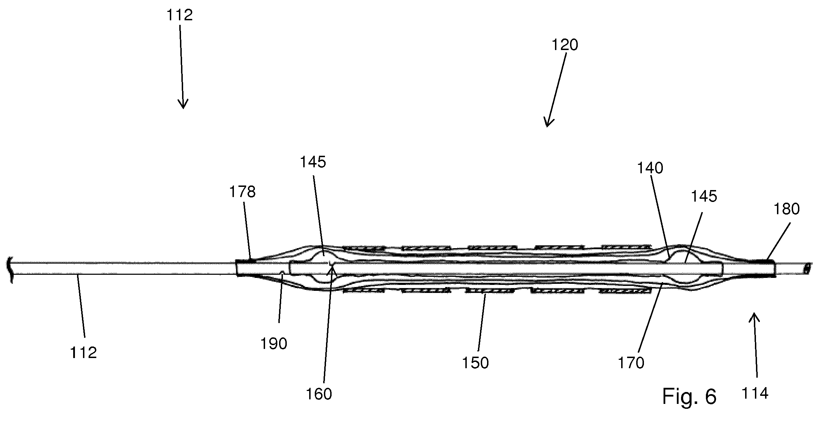

Referring now to Figure 6, this shows an embodiment of balloon catheter apparatus 110 having a double balloon structure 120 which is similar to the double balloon structure of the embodiment of Figures 1 to 5, namely in having an inner balloon 140 disposed within an outer balloon 170 at the distal end 114 of the catheter 112 of the assembly 110. The inner balloon 140 is made of an elastomeric or conformable material, whereas the outer balloon 170 is made of a non-conformable material. The inner and outer balloons 140, 170 have inflated shapes equivalent to the embodiment previously described and shown in Figure 2. The inner and outer balloons 140, 170 are also bonded to the catheter 112 in a manner similar to the embodiment of Figures 1 to 5, the catheter 112 also having the same characteristics as the catheter 12.

As is shown in Figure 5, a stent 150 (or other implantable medical device) is fitted over the balloon structure 120. The stent 150 is of a balloon-expandable type and is shown crimped on the balloon assembly 120 in Figure 6. The balloon structure 120 is longer than the crimped stent 150, such that the balloons 140, 170 extend beyond the extremities of the stent 150.

The outer balloon 170 is shown in its wrapped condition, similar to the condition shown in Figures 4 and 5. The outer balloon 170 can be inflated in order to expand the crimped stent 150 until the latter is pressed against the internal wall surface of a patient's vessel, whereupon the balloon 170 can be deflated, leaving the expanded stent 150 in position.

As is shown in Figure 6, air or other echogenic fluid has been fed into the inner balloon 140, in the manner previously described. The crimped stent 150, however, does not allow the inner balloon 140 to expand to any meaningful extent, as a result of the greater force required to expand the stent 150. However, the fluid pressure in the chamber 42 of the inner balloon 140 causes the balloon 140 to bulge either side of the stent, in two rounded or doughnut-shaped bulges 145, visible in Figure 6. These, it will be appreciated, will be filled with air or other echogenic fluid and result will provide two volumes of echogenic fluid just beyond the two extremities of the crimped stent 150. Thus, when positioned within a patient's lumen, the bulges 145 provide two visible markers under ultrasonic imaging, useful in determining the location of the balloon assembly 120 and in particular of the stent 150 within the vessel so that the latter can be precisely positioned at the treatment site. As the stent 150 will remain crimped on the balloon catheter 110, it will still be possible to move the distal end 114 of the assembly 110 proximally and distally within the vessel. When it is determined that the stent 150 is precisely positioned, the outer balloon 170 can be inflated with suitable inflation fluid, typically saline solution, in order to cause the outer balloon 170 to expand radially outwardly, which in turn causes radial expansion of the stent 150 until the latter is brought into abutment with the internal wall surfaces of the patient's vessel.

As with the first described embodiment, the inner balloon 140 can be left inflated or otherwise deflated during the inflation of the outer balloon 170.

It will be appreciated also that the bulging of the inner balloon 140 helps to retain the stent 150 precisely on the balloon assembly 120 during the initial phase of opening out of the crimped stent 150 and until the inner balloon 170 applies sufficient pressure to hold the opening stent 150 thereto.

The assembly 110 could be used in the deployment of other types of medical device, including, for example, stent grafts and so on.

It will be appreciated that the balloons of the balloon assemblies 20 and 120 are non-porous, so that they each retain fluid in their respective chambers.

It will also be appreciated that the arrangement of concentrically arranged balloons does not necessarily lengthen the balloon structure 20, such that the outer balloon 70, 170 can be chosen to be of a length optimal for the medical treatment to be carried out by the device.

The teachings herein are applicable to PTA balloons. These may have a smooth outer surface but may equally be textured, roughened and/or provided with one or more cutting or scoring elements.

The inflated shapes of the balloons of the balloon assemblies 20 and 120, shown particularly in Figure 2, are examples only and the outer balloon and/or inner balloon could have shapes other than generally cylindrical. Balloons having non-cylindrical shapes are known in the art and can equally benefit from the teachings herein.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in British patent application number 1422607.0, from which this application claims priority, and in the abstract accompanying this application are incorporated herein by reference.

## Claims

1. A balloon catheter assembly including:
a carrier catheter having a distal end and a proximal end, and at least first and second lumens passing through the catheter;
a balloon assembly including an inner inflatable balloon attached in fluid tight manner at the distal end of the carrier catheter, the inner balloon providing an inner chamber; and an outer inflatable balloon disposed concentrically over the inner balloon, the outer balloon being attached in fluid tight manner at the distal end of the catheter and providing an outer chamber;
the first lumen of the catheter including an outlet fluidically coupled to the inner chamber, the second lumen of the catheter including an outlet fluidically coupled to the outer chamber, whereby the inner and outer balloons are independently inflatable;
wherein the inner balloon has an inflated diameter no more than 50% of the inflated diameter of the outer balloon.

2. A balloon catheter assembly according to claim 1, wherein the outer balloon is wrapped over the inner balloon and wherein inflation of the inner balloon does not cause unwrapping of the outer balloon.

3. A balloon catheter assembly according to claim 2, wherein the outer balloon is wrapped over the inner balloon so as to have three to six folded balloon portions wrapped around the carrier catheter.

4. A balloon catheter assembly according to any preceding claim, wherein the inner balloon has an inflated diameter of no more than 2 millimetres.

5. A balloon catheter assembly according to any preceding claim, wherein the inner balloon is longitudinally spaced from the outer balloon on the carrier catheter.

6. A balloon catheter assembly according to any preceding claim, wherein the inner balloon is shorter than the outer balloon.

7. A balloon catheter assembly according to any preceding claim, wherein the inner balloon is longitudinally symmetrically disposed within the outer balloon.

8. A balloon catheter assembly according to any preceding claim, including a source of echogenic fluid coupled to the first lumen, whereby the inner balloon is inflated with said echogenic fluid.

9. A balloon catheter assembly according to claim 8, wherein the fluid is air.

10. A balloon catheter assembly according to any preceding claim, wherein the inner balloon is wholly contained in the outer balloon.

11. A balloon catheter assembly according to claim 10, wherein the inner balloon is attached to the carrier catheter at attachment points, the attachment point being disposed within the outer chamber of the outer balloon.

12. A balloon catheter assembly according to any preceding claim, wherein the inner balloon is made of polyurethane or polyether block amide.

13. A balloon catheter assembly according to any preceding claim, including an implantable medical device carried on the balloon assembly.

14. A balloon catheter assembly including:
a carrier catheter having a distal end and a proximal end, and at least first and second lumens passing through the catheter;
a balloon assembly including an inner inflatable balloon attached in fluid tight manner at the distal end of the carrier catheter, the inner balloon providing an inner chamber; and an outer inflatable balloon disposed concentrically over the inner balloon, the outer balloon being attached in fluid tight manner at the distal end of the catheter and providing an outer chamber;
the first lumen of the catheter including an outlet fluidically coupled to the inner chamber, the second lumen of the catheter including an outlet fluidically coupled to the outer chamber, whereby the inner and outer balloons are independently inflatable;
wherein the outer balloon is wrapped over the inner balloon prior to inflation and wherein inflation of the inner balloon does not cause unwrapping of the outer balloon.

15. A balloon catheter assembly including:
a carrier catheter having a distal end and a proximal end, and at least first and second lumens passing through the catheter;
a balloon assembly including an inner inflatable balloon attached in fluid tight manner at the distal end of the carrier catheter, the inner balloon providing an inner chamber; and an outer inflatable balloon disposed concentrically over the inner balloon, the outer balloon being attached in fluid tight manner at the distal end of the catheter and providing an outer chamber;
the first lumen of the catheter including an outlet fluidically coupled to the inner chamber, the second lumen of the catheter including an outlet fluidically coupled to the outer chamber, whereby the inner and outer balloons are independently inflatable;
and a source of air connected to the first catheter lumen for inflating the inner balloon with air.
